# EUROPEAN PATENT APPLICATION

(11) **EP 4 421 162 A1**
(43) Date of publication of application: **28.08.2024**
(21) Application number: 22880937.2
(22) Date of filing: 07.10.2022

(54) **AUTOIMMUNE DISEASE THERAPEUTIC AGENT INCLUDING OLIGONUCLEOTIDE THAT SELECTIVELY BINDS TO IFN-gamma, AND SAID OLIGONUCLEOTIDE**

(30) Priority: 11.10.2021 JP 2021166794
(71) Applicant: TAGCyx Biotechnologies Inc., Tokyo 1530041 (JP)
(72) Inventor: HARADA, Kaori, Tokyo 153-0041 (JP); HORI, Miyuki, Tokyo 153-0041 (JP)
(74) Representative: Schön, Christoph
(86) International application number: PCT/JP2022/037560
(87) International publication number: WO 2023/063242

(57) **Abstract**

A purpose of the present invention is to provide an autoimmune disease therapeutic agent that can selectively inhibit IFN-γ, has no risk of biological contamination, and can be stored at room temperature. Provided is an autoimmune disease therapeutic agent containing as an active ingredient a DNA oligonucleotide having a nucleotide sequence set forth in any of SEQ ID NO:1 to 3 and selectively binding to IFN-γ. In another aspect, provided is a DNA oligonucleotide having a nucleotide sequence set forth in any of SEQ ID NO:1 to 3 and having a therapeutic effect on autoimmune diseases by selectively binding to IFN-γ.

## Description

### [Technical Field]

The present invention relates to an autoimmune disease therapeutic agent containing an oligonucleotide that selectively binds to interferon-γ(IFN-γ), and the oligonucleotide.

### [Background Art]

Alopecia areata is a widespread hair loss condition that occurs widely regardless of age or gender, and frequently occurs on the head. Recent studies have revealed that autoimmune abnormalities are involved in the pathogenesis of alopecia areata, and nowadays alopecia areata is recognized as one of the autoimmune diseases. Normal hair follicle tissues and the surrounding areas are in an immune-tolerant environment, with little or no expression of MHC class I and II. In contrast, in alopecia areata lesions, expression of MHC class I and II increases and immune tolerance of hair follicles is broken.

It is known that in tissues at the site of hair loss caused by alopecia areata, activated lymphocytes (mainly CD8-positive cytotoxic T-cells) that accumulate around the hair follicle recognize and attack autoantigens in the hair root and this reaction has been verified by various scientific studies (Non Patent Literature 1). These activated lymphocytes are shown to overproduce interferon-γ (IFN-γ), and IFN-γ is known to be a factor in the development of alopecia areata. IFN-γ induces expression of MHC class I and II in the proximal outer root sheath and hair matrix cells and breaks immune tolerance in the hair follicle. In addition, expression of MHC class II of dendritic cells increases around the hair follicle in alopecia areata, and these increased expressions of MHC class I and II are linked to the development of alopecia areata.

IFN-γ is known as a major causative cytokine in the pathogenesis of autoimmune diseases (Non Patent Literature 2) and nowadays recognized as one of the important targets for treating autoimmune diseases. Alopecia areata is postulated to be caused when overproduced IFN-γ in the hair follicle and the surrounding areas binds to cell membrane receptors to transmit signals within cells via activation of Janus kinase, thereby overexpressing MHC class I and II. By inhibiting Janus kinase activity, hair growth was shown (Non Patent Literature 3), suggesting that the treatment of alopecia areata by inhibiting activity of IFN-γ as a target molecule may be useful. In clinical researches in which anti-IFN-γ antibodies were administered to patients with autoimmune diseases such as alopecia areata, rheumatoid arthritis and multiple sclerosis in order to neutralize IFN-γ activity, improvement in symptoms have been observed (Non Patent Literature 4).

### [Citation List]

### [Non Patent Literature]

[NPL 1] Jillian F. Rork, et. al., Curr. Opin. Pediatr., 2016, August; 28(4), 463-469.
[NPL 2] Simon Skurkovich, et. al., Expert Rev. Clin. Immunol., 2005, 1(1), 11-25.
[NPL 3] Aniseh Samadi, et. al., Journal of Dermatological Treatment, 2017, 28(6), 476-483.
[NPL 4] B. Skurkovich., et. al., Ernst Schering Res. Found Workshop, 2006, 56, 1-27.

### [Summary of Invention]

### [Technical Problem]

As described above, inhibitors of IFN-γ activity are likely to be promising as therapeutic agents for autoimmune diseases. Previously, as agents that inhibit IFN-γ activity, antibodies and Janus kinase inhibitors have been developed. However, anti-IFN-γ antibodies, for example, have some problems, including that (1) since they are biologics, there is a risk of biological contamination and the like, (2) in case of heterologous antibodies, antigenicity becomes a problem in long-term administration, and (3) since they are protein preparations, a cold chain is required for storage and transportation.

In addition, with regard to the problem (2) above, the antibody production rate for common antibody drugs is said to be about 30%. Therefore, when a long-term treatment is required, a case is often observed in which an antibody against another antibody is generated, causing an anaphylactic reaction and making it difficult to continue the treatment.

Furthermore, with regard to the problem (1) above, since serum and other substances are often used in the producing process of biologics, there is concern about the potential risk of contamination by viruses and other contaminants. With regard to the problem (3), the protein preparation always requires handling at a low temperature, which increases the cost of transportation and storage, and also reduces convenience for patients using the preparation.

Four Janus kinase inhibitors including Tofacitinib (product name: Xeljanz^{®}), Baricitinib (product name: Olmient^{®}), Peficitinib (product name: Smyraf^{®}) and Upadacitinib (product name: RINVOQ^{®}) have been applied to and marketed for rheumatoid arthritis, an autoimmune disease. Since these Janus kinase inhibitors are low-molecular-weight compounds that can be produced by chemical synthesis, they are postulated not to have the problems caused by being antibodies described above. On the other hand, multiple subtypes of Janus kinase exist and are activated by binding to the intracellular domains of multiple cytokine receptors including interleukin 2 (IL-2) receptor, interleukin 4 (IL-4) receptor, interleukin 7 (IL-7) receptor, and interferon-α (IFN-α) receptor, as well as the IFN-γ receptor, and transmit receptor signals.

Therefore, Janus kinase inhibitors may inhibit signaling of not only IFN-γ but also IL-2, IL-4, IL-7, IFN-α, and the like (Yvan Jamilloux, et. al., Autoimmunity Reviews, 2019, 18, 102390). This raises safety concerns for long-term administration and suggests a possibility of developing unanticipated adverse effects.

The present invention was made in view of these circumstances, and has the purpose of providing an autoimmune disease therapeutic agent that can selectively inhibit IFN-γ, has no risk of biological contamination, has no problem of antigenicity in long-term administration, and can be stored at room temperature.

### [Solution to Problem]

In order to solve the problems described above, the autoimmune disease therapeutic agent containing as an active ingredient an oligonucleotide that selectively binds to IFN-γ and the oligonucleotide of the present invention adopt the following aspects.

The first aspect of the present invention provides an autoimmune disease therapeutic agent containing as an active ingredient a DNA oligonucleotide having a nucleotide sequence set forth in any of SEQ ID NO:1 to 3 and selectively binding to interferon-γ (IFN-γ). The DNA oligonucleotide of this aspect exerts a therapeutic effect on autoimmune diseases by selectively binding to IFN-γ.

The nucleotide sequence set forth in SEQ ID NO:2 is a sequence in which an oligonucleotide comprising natural bases of 9 residues is added to a 3' end of the nucleotide sequence set forth in SEQ ID NO:1.

The nucleotide sequence set forth in SEQ ID NO:3 is a sequence in which a 53rd base from the 5' end of the nucleotide sequence set forth in SEQ ID NO:2 was replaced with any base.

In the first aspect described above, a base X in a sequence of the DNA oligonucleotide having the nucleotide sequence set forth in SEQ ID NO:3 may be an artificially produced base, and the artificially produced base may be chemically modified with a low-molecular-weight compound.

The low-molecular-weight compound in the first aspect described above may be an anti-inflammatory compound selected from glucocorticoid, tacrolimus, sirolimus, cyclosporine, methotrexate and leflunomide, or an antibiotic selected from erythromycin, azithromycin, kanamycin, ofloxacin, gatifloxacin, tetracycline and vancomycin.

In the first aspect described above, a base X in a sequence of the DNA oligonucleotide having the nucleotide sequence set forth in SEQ ID NO:3 may be an artificially produced base, and the artificially produced base may be chemically modified with a medium-molecular-weight compound, a high-molecular-weight compound, a biopolymer, or a biocompatible polymer.

The medium-molecular-weight compound in the first aspect described above may have a molecular weight of about 1000 to 20000. The high-molecular-weight compound of the present aspect may have a molecular weight of about 20000 to 400000.

The high-molecular-weight compound in the first aspect described above may be any biocompatible large molecule with a molecular weight of 20000 or more. Examples of medium-molecular-weight compounds or high-molecular-weight compounds in the present aspect include, but are not limited to, polyethylene glycols (PEGs), bipolar polymers, oligosaccharides, fat-soluble polymers, peptides, oligonucleotides, and antibodies. Antibodies belong to high-molecular-weight compounds, while PEGs, bipolar polymers, oligosaccharides, fat-soluble polymers, peptides, and oligonucleotides belong to medium-molecular-weight compounds or high-molecular-weight compounds, depending on their molecular weight. The molecular weight of a medium-molecular-weight compound or a high-molecular-weight compound is expressed as an average molecular weight defined by the number average molecular weight (Mn) or the weight average molecular weight (Mw).

In the first aspect described above, the autoimmune disease to be treated may be an autoimmune skin disease selected from the group consisting of alopecia areata, vitiligo vulgaris, psoriasis, scleroderma, dermatomyositis, atopic dermatitis, cutaneous lupus erythematosus and IgA-related dermatitis.

In the first aspect described above, the autoimmune disease to be treated may be an autoimmune disease in the bladder selected from the group consisting of interstitial cystitis and bladder pain syndrome.

In the first aspect described above, the autoimmune disease to be treated may be an autoimmune disease in the eye selected from the group consisting of uveitis, dry eye, keratitis sicca, episcleritis and scleritis.

In the first aspect described above, the autoimmune disease to be treated may be a systemic autoimmune disease selected from the group consisting of multiple sclerosis, systemic lupus erythematosus, endometriosis, myocarditis, type I diabetes mellitus, thyroiditis, premature ovarian failure, Sjogren syndrome, Raynaud syndrome, rheumatoid arthritis, myasthenia gravis, Takayasu arteritis, Addison disease, Guillain-Barre syndrome, hypothyroidism, sarcoidosis, hemophagocytic lymphohistiocytosis, thrombotic thrombocytopenic purpura, and autoimmune hepatitis.

In the first aspect of the present invention described above, the autoimmune disease to be treated may be an autoimmune disease of the digestive organs selected from the group consisting of Crohn disease, ulcerative colitis, autoimmune pancreatitis, biliary cholangitis, and autoimmune atrophic gastritis.

The second aspect of the present invention provides a DNA oligonucleotide having a nucleotide sequence set forth in any of SEQ ID NO:1 to 3 and having a therapeutic effect on autoimmune diseases by selectively binding to interferon-γ (IFN-γ). The DNA oligonucleotide of this aspect exerts a therapeutic effect on autoimmune diseases by selectively binding to IFN-γ. The nucleotide sequence set forth in SEQ ID NO:2 is the nucleotide sequence set forth in SEQ ID NO:1 having an oligonucleotide of 9 natural base residues added to the 3' end. The nucleotide sequence set forth in SEQ ID NO:3 is a sequence in which the 53rd base counted from the 5' end of the nucleotide sequence set forth in SEQ ID NO:2 was changed to any base X, and the X is any natural base or artificially produced base. The artificially produced base of this aspect in a sequence of the DNA oligonucleotide having a nucleotide sequence set forth in SEQ ID NO:3 may be chemically modified with the low-molecular-weight compound, the medium-molecular-weight compound, or the high-molecular-weight compound described above.

The autoimmune diseases for which the autoimmune disease therapeutic agent of the second aspect above is given for the treatment may be the autoimmune skin diseases, the autoimmune diseases in the bladder, the autoimmune diseases in the eye, the systemic autoimmune diseases, and the autoimmune diseases of the digestive organs exemplified above.

### [Advantageous Effects of Invention]

According to the DNA oligonucleotide having a therapeutic effect on autoimmune diseases of the present invention, a selective inhibition of IFN-γ is achieved by selectively binding to IFN-γ. Also, according to the DNA oligonucleotide having a therapeutic effect on autoimmune diseases of the present invention, its production is made without a risk of biological contamination, since there is no need to use serum or other substances in its production. Further, the DNA oligonucleotide having a therapeutic effect on autoimmune diseases of the present invention can be stored at room temperature, which is advantageous compared to conventional methods in terms of transportation and storage costs and can improve convenience for patients who use it.

According to the autoimmune disease therapeutic agent containing the DNA oligonucleotide as an active ingredient of the present invention, the DNA oligonucleotide of the present invention inhibits only the action of IFN-γ, even when administered for a long time, thus reducing unanticipated adverse effects compared to Janus kinase inhibitors and other therapeutic agents. Further, when compared to anti-IFN-γ antibodies, the DNA oligonucleotide of the present invention is less antigenic than antibodies, which makes it an agent that can be used for a long period of time. Furthermore, the DNA oligonucleotide of the present invention has no risk of biological contamination and can be stored at room temperature.

### [Brief Description of Drawings]

[Fig. 1] Fig. 1 shows a change in the number of hairs on a grafted skin tissue piece before and after administration of the DNA oligonucleotide of an embodiment of the present invention. The vertical axis shows the change in the number of hairs per grafted skin tissue piece.
[Fig. 2A] Fig. 2A shows an inhibition of MHC class I expression in the dermal sheath cup by administration of the DNA oligonucleotide according to an embodiment of the present invention.
[Fig. 2B] Fig. 2B shows an inhibition of MHC class I expression in the outer root sheath by administration of the DNA oligonucleotide according to an embodiment of the present invention.
[Fig. 3A] Fig. 3A shows an inhibition of MHC class II expression in the connective tissue sheath by administration of the DNA oligonucleotide according to an embodiment of the present invention.
[Fig. 3B] Fig. 3B shows an inhibition of MHC class II expression in the outer root sheath by administration of the DNA oligonucleotide according to an embodiment of the present invention.

### [Description of Embodiments]

It is known that overproduction of IFN-γ, which is a factor in the development of alopecia areata, induces expression of major histocompatibility complex (MHC) class II on T cell membranes (Viktor Steimle, et. al, Science, 1994, 265(5168), 106-109; Carmen Gianfrani, et. al., Journal of Autoimmunity, 2018 (89), 1-10; Giulio Cavalli, et. al., Proc. Natl. Acad. Sci. USA, 2016, 113(5), 1363-1368), and further inhibits differentiation of regulatory T cells (Treg cells) that control immunity in an inhibitory manner (Susan A. Alalehan, et. al., Sur. J. Immunol., 2015, 45, 988-998). The mechanism above is postulated to be one of the main causes of the development of autoimmune diseases. Therefore, IFN-γ is recognized as one of the important targets for treating autoimmune diseases. Emapalumab, an anti-IFN-γ antibody, was approved by FDA in 2018 for hemophagocytic lymphohistiocytosis, an autoimmune disease that is as refractory as the diseases described above, and is marketed under the trade name Gamifant.

However, as described above, anti-IFN-γ antibodies have problems such as biological contamination and other risks due to being biologics, antigenicity in long-term administration, and conditions for storage and transportation due to being protein preparations. Therefore, the creation of a therapeutic agent that can solve these problems and be effective is desired.

The present inventors attempted to develop an IFN-γ inhibitor using a DNA aptamer as a means to solve the problems described above. The DNA aptamer is a ligand molecule which forms a secondary structure or a tertiary structure of a single-stranded DNA oligonucleotide by forming a complementary strand between complementary sequences in the DNA oligonucleotide molecule, and binds specifically and strongly to a target molecule by its steric structure. The binding of the DNA aptamer can inhibit, suppress or enhance activity of the target molecule. Although DNA aptamers are smaller than antibodies, with a molecular weight of about one-tenth or less than that of antibodies, they have high compatibility and high target selectivity comparable to those of antibodies. Therefore, it is postulated that an IFN-γ inhibitor using a DNA aptamer may be a suitable modality as a means to solve the problem, since the occurrence of adverse effects due to off-target can be minimized and DNA aptamers can be produced through chemical synthesis.

DNA aptamers have the following advantages and are expected to be useful: (1) with a relatively small molecular weight, administration by transdermal formulations such as ointments and patches may be possible; (2) as chemically synthesized products, a risk of biological contamination is low; (3) generally antigenicity is low; (4) due to being DNA, sufficient stability is secured at room temperature under nuclease-free and neutral conditions; and (5) with almost no inhibitory activity to cytochrome P450, a drug-metabolizing enzyme, there is no effect on concomitant agents, and the like. Further, DNA aptamers do not have a problem that an antibody is generated against another antibody, the problem that occurs when long-term treatment with the antibody is necessary, and therefore, long-term administration is possible.

The DNA oligonucleotide of the present embodiment can be used as a DNA aptamer. As a specific means to treat diseases using the DNA aptamer, the means is assumed to treat autoimmune diseases by neutralizing IFN-γ through administration of the aptamer itself or a modified form of the aptamer. In the present embodiment, DNA oligonucleotides listed in Table 1 are used as DNA aptamers. The present embodiment comprises the use of the DNA oligonucleotides having the nuclear acid sequences listed in Table 1 as autoimmune disease therapeutic agents.

**[Table 1]**

| SEQ ID NO: | Sequence (5' to 3') |
|---|---|
| 1 | CCCGCCCGGGTCCGCGAAGCGGTAGGTDsTGGGCTAGGCDsGCTGGCGGG |
| 2 | CCCGCCCGGGTCCGCGAAGCGGTAGGTDsTGGGCTAGGCDsGCTGGCGGGCGCGAAGCG |
| 3 | CCCGCCCGGGTCCGCGAAGCGGTAGGTDsTGGGCTAGGCDsGCTGGCGGGCGCGXAGCG |

The sequence set forth in SEQ ID NO:2 of Table 1 is a sequence in which an oligonucleotide comprising natural bases of 9 residues is added to the 3' end of the sequence set forth in SEQ ID NO:1. The sequence set forth in SEQ ID NO:3 of Table 1 is a sequence in which the 53rd base from the 5' end of the sequence set forth in SEQ ID NO:2 was replaced with any base X. The X represents any natural base, any non-natural base or a modified base, or a modified base to which a low-molecular-weight compound, a peptide, an oligonucleic acid, an oligosaccharide, a protein or the like, or a high-molecular-weight compound used in vivo (biopolymer) or a biocompatible polymer is bound.

Examples of high-molecular-weight compounds include polyethylene glycols (PEG) with a molecular weight of 20000 or more and any biocompatible large molecules with a molecular weight of 20000 or more. A biocompatible polymer is a chemically synthesized compound that is not normally used in vivo and safe enough to be placed in vivo without causing inflammation or toxic reactions. Examples of medium-molecular-weight compounds include peptides, oligonucleic acids, oligosaccharides, proteins, PEGs, and any biocompatible polymers with a molecular weight of 1000 or more and less than 20000. Examples of low-molecular-weight compounds include antibiotics with a molecular weight of about 200 to 1000.

As functional groups for modification, azido group (-N₃), amino group (-NH₂), carboxyl group (-COOH) or its active ester, alkynyl group (-CC) or a cyclic structure having an alkynyl structure, formyl group (-CHO), hydrazide group (-NH-NH₂), hydroxyl group (-OH), thiol group (-SH), cyano group (-CN), vinyl group (-CHCH₂) and maleimide group can be used.

As used herein, "natural base" refers to either adenine, guanosine, cytosine, or thymine. As used herein, "non-natural base" refers to a base that is artificially synthesized having properties similar to the natural base, and is sometimes referred to as "artificial base" herein. As used herein, "modified base" refers to a base to which a side chain structure with one or more functional groups activated for modification is added, and is a kind of "artificially produced base". Examples of modifications include methylation, deamination, atomic place exchange, thiolation of oxygen in phosphate site, and introduction of a water-soluble or fat-soluble substituent group into the base portion of a natural base. Specifically, the examples include modified pyrimidines, modified purines, and other heterocyclic bases. Ds in SEQ ID NO:1 to 3 of Table 1 represents 7-(2-thienyl)imidazo[4,5-b]pyridine, an artificial base. As an artificial base, in addition to Ds itself, a base with a side chain introduced into Ds may be used. Hereafter, in the present embodiment, a DNA aptamer having a sequence set forth in SEQ ID NO:1, 2, or 3 of Table 1 will be referred to as "Aptamer 1", "Aptamer 2", or "Aptamer 3", respectively.

In the present embodiment, it was observed that when a DNA aptamer listed in Table 1 (Aptamer 2) was injected intradermally into the transplanted skin of an immune-tolerance mouse of autoimmune hair loss model which was transplanted with human skin tissue piece, hair loss was suppressed and regeneration of hair once lost was promoted (Example 4). Details will be described later.

Pathological analysis of the mechanism of activity expression of Aptamer 2 or Aptamer 3 in this model revealed that Aptamer 2 or Aptamer 3 almost completely inhibited expression of MHC class I and II. Detailed results will be described later. That is, it is postulated that by inhibiting activity of IFN-γ, Aptamer 2 or Aptamer 3 inhibited production of MHC class I and II, the source of expression of autoimmunity, to improve autoimmunity, thus improving the symptoms of alopecia areata. This indicates that Aptamer 2 or Aptamer 3 is useful as a therapeutic agent not only for alopecia areata but also for other autoimmune diseases (Vasiliki Matzaraki, et. al, Genome Biology, 2017, 18:76; Giulio Cavalli, et. al, Proc. Natl. Acad. Sci. USA, 2016, 113(5), 1363-1368), and that providing an IFN-γ inhibitor by means of DNA aptamers could be a means to solve the problem.

As a DNA aptamer, DNA oligonucleotides having any of the sequences listed in Table 1 can be used as they are,
or it is also possible to use those modified at a site that does not affect activity of the DNA aptamer. Examples of modified forms of DNA aptamers include DNA aptamers bound to medium- or high-molecular-weight compounds such as PEGs, peptides, and oligonucleotides by a chemical method, multimerized DNA aptamers by a chemical method derived from same DNA aptamers, and DNA aptamers in which their sequences are partially converted or modified. When a DNA oligonucleotide of the present embodiment is modified and used as a DNA aptamer, the base portion is preferred as the modification portion. An artificial base or a modified base can be modified using existing methods, and the 3' and 5' ends can also be modified.

As drug formulations for systemic administration, formulations can be prepared as an injectable formulation in vials containing lyophilized powders, vials containing aptamer solution, and pre-filled syringes.

The DNA aptamer of the present embodiment can be produced as a preparation for inhalation by placing a nanoparticle adsorbing or containing the DNA aptamer or a solution thereof, or a powder of the DNA aptamer granulated to an appropriate size with a granulating material in an inhalation device.

The DNA aptamer of the present embodiment can be used as an eye drop by dissolving it as it is utilizing its high water-solubility.

As preparations for injection, preparations for inhalation and eye drops, it is possible to use them in a form that the DNA aptamer of the present embodiment is encapsulated or bonded in nanoparticles such as fatty nanoparticles, nanoparticles of biodegradable polymers such as PLGA (Polylactic-co-Glycolic Acid), gold nanoparticles, and then dispersed or dissolved in physiological saline solution, physiological buffer solution, and the like.

The DNA aptamer of the present embodiment can be applied as a topical transdermal agent such as a solution, an ointment, a cream, a lotion, a milky lotion, an emulsion, a gel, a biodegradable microneedle, or a poultice.

In the process of producing a transdermal administration formulation, as an absorption enhancer, lower alcohols such as ethanol, polyhydric alcohols such as ethylene glycol, fatty acids, esters such as ethyl acetate, surfactants, and ionic liquids and the like may be used. For the production of the transdermal administration formulation, production process in which biodegradable polymers such as polylactic acid or liposomes are used for making nanoparticles is applicable, and these processes can be combined as appropriate depending on purposes.

The DNA aptamer of the present embodiment can also be used as an administration preparation using a device compatible with physical transdermal absorption enhancement methods such as Iontophoresis, Electroporation, Thermalporation, Sonophoresis, a Microneedle array patch, a Needleless syringe, and a micropump.

### [Examples]

### Example 1: Synthesis of DNA aptamer

Aptamer 1 and aptamer 2 were chemically synthesized by the methods described in PCT International Publication No. WO 2013/073602 and PCT International Publication No. WO 2016/143700.

### Example 2: Example of synthesis of aptamer 3

Aptamer 3 was synthesized by introducing Amino-Modifier C6-dT Amidite to the position of X in the sequence of SEQ ID NO:3 using the methods described in PCT International Publication No. WO 2013/073602 and PCT International Publication No. WO 2016/143700. For other substituents of X, they can be synthesized by using commercially available artificial base or modified base amidites.

### Example 3: Synthesis of PEG-modified DNA aptamer

With the base of X produced in Example 2, Aptamer 3 (1 eq) having a primary amine side chain and commercially available NHS-PEG (40000) (1.5 eq) were mixed in a phosphate buffer at pH 7 to 8 and the mixture was stirred for 1 day at room temperature. The reaction solution was concentrated and the resulting modified form was purified by reversed phase HPLC to obtain a PEG-modified form of Aptamer 3. It was confirmed that the obtained PEG-modified form of Aptamer 3 retained its ability to bind to IFN-γ using a SPR (Surface Plasmon Resonance).

PEG-modified forms of DNA aptamers are utilized for improving Pharmacokinetics (PK)-Pharmacodynamics (PD) Profile that is commonly used for proteins, peptides, oligonucleic acids including aptamers to achieve improved pharmacokinetics, and a number of PEG-modified aptamers have been developed. It is known that if binding activity of a PEG-modified aptamer to a target protein is retained, it shows the same level of activity in the body as the aptamer before PEG modification, and there is almost no toxic expression due to the PEG modification (C. Simone Fishburn, Journal of Pharmaceutical Sciences, 2008, 97, 10, 4167-4183; Katarina D. Kovacevic, et. al., Advanced Drug Delivery Reviews, 2018, 134, 36-50).

### Example 4: Confirmation of therapeutic effect using humanized model mouse with alopecia areata

### Step 1 Creation of humanized model mouse

A humanized model mouse with alopecia areata induced by an intradermal injection of human activated lymphocytes into the transplanted skin was created based on the method described in A. Gilhar, et. al., Journal of Investigative Dermatology, 2013 (133), 844-847.

### Step 2

The humanized model mice created were divided into three groups, and each group was treated with Vehicle (PBS), Dexamethasone + Minoxidil (Positive control), and Aptamer 2. To the Vehicle group, 15 µL of PBS was administered to the transplanted skin once every 2 days. To the group treated with Aptamer 2 (hereafter referred to as the "aptamer-treated group"), a 15 µL of aptamer solution in PBS was administered to the transplanted skin once every 2 days, and the concentration of the Aptamer 2 solution was gradually increased from 12 nM to 300 nM over 143 days. To the group treated with Dexamethasone + Minoxidil, 2 mg of Dexamethasone and 40 µL of 5% Minoxidil were applied to the transplanted skin once daily.

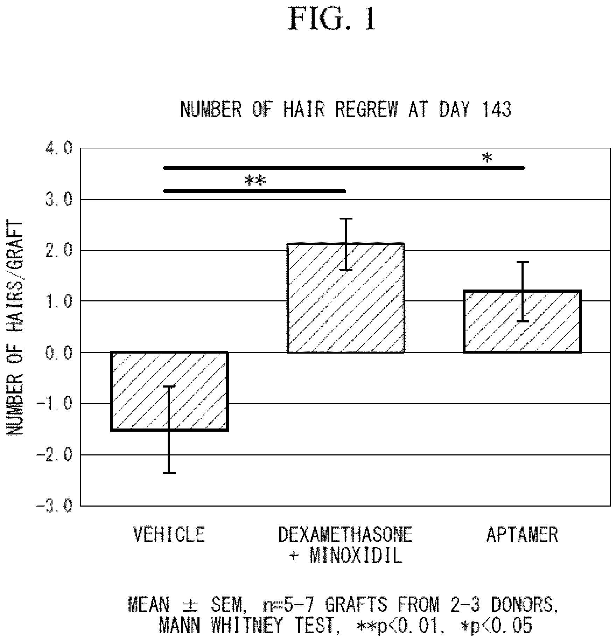

The results after administration in Example 4 are shown in Fig. 1. Fig. 1 shows a change in the number of hairs on the grafted skin tissue piece before and after the administration, with the vertical axis showing the change in the number of hairs per grafted skin tissue piece. In the Vehicle group ("Vehicle" in Fig. 1), further hair loss progressed during the PBS administration period, while in the Positive control group ("Dexamethasone + Minoxidil" in Fig. 1) and the Aptamer-treated group ("Aptamer" in Fig. 1), further hair loss was prevented and also hair regeneration was observed.

Pathological analysis results of the area around the hair root tissue showed a significant inhibition of CD8-positive T-cell infiltration in the Positive control group and the Aptamer-treated group. This suggests that in the Positive control group and the Aptamer-treated group, inhibition of inflammatory reaction suppressed hair loss progression and promoted hair regeneration.

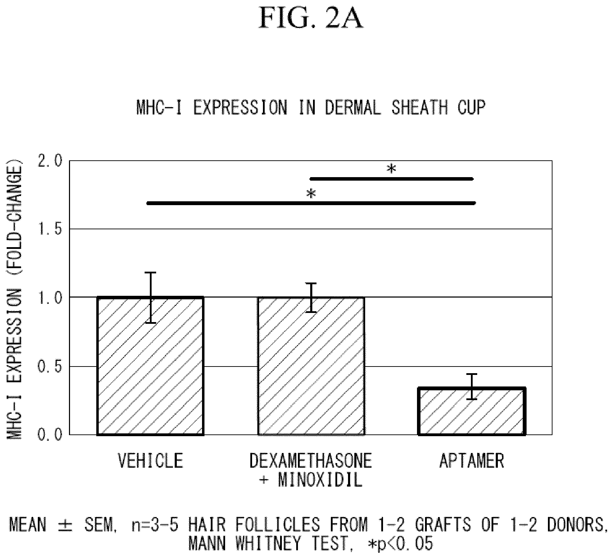

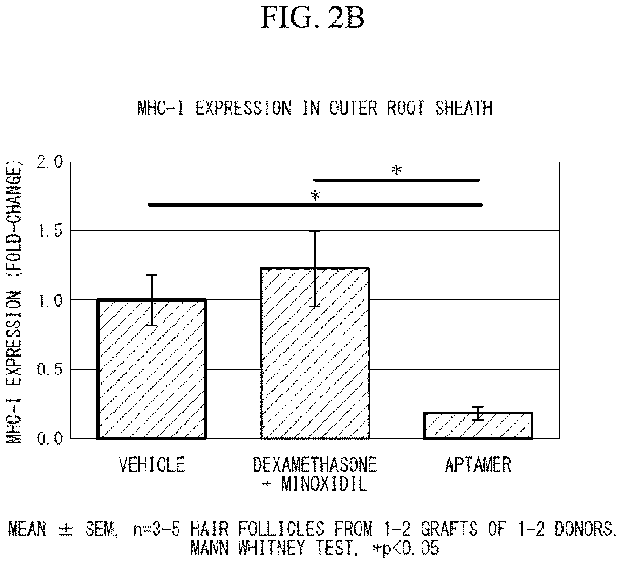

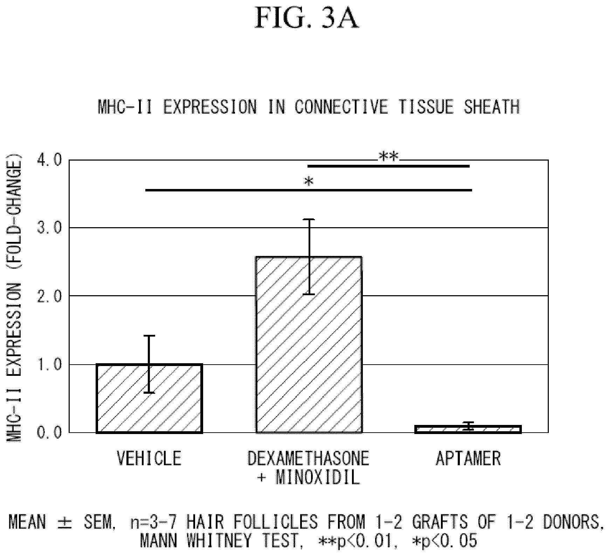

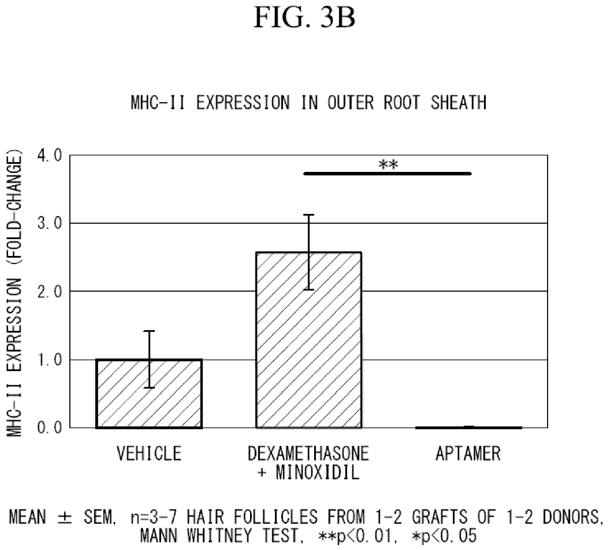

Further, expression of MHC in the tissues surrounding the hair root after the administration in Example 4 was examined. The results for MHC class I are shown in Fig. 2A and Fig. 2B, and the results for MHC class II are shown in Fig. 3A and Fig. 3B, respectively. The vertical axis shows the expression levels of MHC class I (Fig. 2A and Fig. 2B) or II (Fig. 3A and Fig. 3B) as relative values to the expression level in the respective Vehicle group ("Vehicle" in Fig. 2A, Fig. 2B, Fig. 3A and Fig. 3B) as 1.

Inhibition of expression of both MHC class I and II was observed only in the Aptamer-treated group ("Aptamer" in Fig. 2A, Fig. 2B, Fig. 3A and Fig. 3B). This indicates that the mechanism that suppressed inflammation and promoted hair regeneration was different between the Positive control group ("Dexamethasone + Minoxidil" in Fig. 2A, Fig. 2B, Fig. 3A and Fig. 3B) and the Aptamer-treated group. It is assumed that the Positive control group showed direct suppression of inflammation by activation of glucocorticoid receptors, while the Aptamer-treated group suppressed inflammation by inhibiting the production of MHC class I and II, which cause autoimmunity. In other words, in the Aptamer-treated group, hair follicle tissues recovered from the breakdown of immune tolerance, suggesting that a more fundamental therapeutic effect was obtained. This result suggests that the DNA aptamer of the present embodiment may be able to suppress inflammatory reactions not only in autoimmune skin diseases, but also in autoimmune diseases that occur in other tissues, such as the eye and the bladder, through similar mechanisms.

It was confirmed that the use of the therapeutic agent containing the DNA aptamer of the present embodiment can prevent hair loss and also regenerate hair. Further, as a result of the pathological analysis, it was confirmed that the administration of the DNA aptamer of the present embodiment can almost completely inhibit expression of MHC class I and II.

It is postulated that the results in Example 4 above may have resulted from the strong inhibition of IFN-γ activity by the DNA aptamer of the present embodiment. Therefore, the DNA aptamer of the present embodiment can be used to provide the most unprecedented and effective therapeutic agent and treatment method for autoimmune diseases, including alopecia areata.

From the results of the above Examples, it is also postulated that the DNA aptamer of the present embodiment binds to IFN-γ with high specificity. When compared to Janus kinase inhibitors (Yvan Jamilloux, et. al., Autoimmunity Reviews, 2019, 18, 102390), which by the pharmacological nature inevitably inhibit multiple cytokine signals, the aptamer of the present embodiment, which selectively inhibits IFN-γ activity, can reduce the likelihood of development of adverse effects.

DNA aptamers are generally less likely to produce anti-DNA aptamer antibodies. This allows the DNA aptamer of the present embodiment to be administered over a long period of time in the treatment of autoimmune diseases.

The DNA aptamer of the present embodiment can be produced completely through chemical synthesis. Therefore, it can be provided as a safe agent with stable quality and low risk of biological contamination.

The DNA aptamer of the present embodiment can be produced at a lower cost than biologics. Further, while biologics require low-temperature conditions for their storage, DNA aptamers are stable at room temperature. Therefore, a cold chain is not necessarily required in the transportation and storage of the drug formulation containing the DNA aptamer of the present embodiment.

By making the DNA aptamer of the present embodiment into a transdermal administration formulation, a therapeutic agent which is not invasive in administration, has a low risk of adverse effects and is easy to use can be provided.

The therapeutic agent containing the DNA aptamer of the present embodiment can be indicated for systemic autoimmune diseases by systemically administering it as an injectable formulation. Further, when made into an injectable formulation, it can be a drug formulation which is easy to use for patients, such as a pre-filled syringe which can be stored at room temperature.

## Claims

1. An autoimmune disease therapeutic agent comprising as an active ingredient a DNA oligonucleotide having a nucleotide sequence set forth in any of SEQ ID NO:1 to 3 and selectively binding to interferon-γ (IFN-γ).

2. The autoimmune disease therapeutic agent according to claim 1, wherein a base X in a sequence of the DNA oligonucleotide having a nucleotide sequence set forth in SEQ ID NO:3 is an artificially produced base, and the artificially produced base is chemically modified with a low-molecular-weight compound.

3. The autoimmune disease therapeutic agent according to claim 2, wherein the low-molecular-weight compound is an anti-inflammatory compound selected from glucocorticoid, tacrolimus, sirolimus, cyclosporine, methotrexate and leflunomide, or an antibiotic selected from erythromycin, azithromycin, kanamycin, ofloxacin, gatifloxacin, tetracycline and vancomycin.

4. The autoimmune disease therapeutic agent according to claim 1, wherein a base X in a sequence of the DNA oligonucleotide having a nucleotide sequence set forth in SEQ ID NO:3 is an artificially produced base, and the artificially produced base is chemically modified with a medium-molecular-weight compound, a high-molecular-weight compound, a biopolymer, or a biocompatible polymer.

5. The autoimmune disease therapeutic agent according to claim 4, wherein the high-molecular-weight compound is a polyethylene glycol (PEG) with a molecular weight of 20000 or more or any biocompatible large molecule with a molecular weight of 20000 or more.

6. A DNA oligonucleotide having a nucleotide sequence set forth in any of SEQ ID NO:1 to 3 and having a therapeutic effect on autoimmune diseases by selectively binding to interferon-γ (IFN-γ).

7. The DNA oligonucleotide having a therapeutic effect on autoimmune diseases according to claim 6, wherein a base X in the nucleotide sequence set forth in SEQ ID NO:3 is an artificially produced base, and the artificially produced base is chemically modified with a low-molecular-weight compound.

8. The DNA oligonucleotide having a therapeutic effect on autoimmune diseases according to claim 7, wherein the low-molecular-weight compound is an anti-inflammatory compound selected from glucocorticoid, tacrolimus, sirolimus, cyclosporine, methotrexate and leflunomide, or an antibiotic selected from erythromycin, azithromycin, kanamycin, ofloxacin, gatifloxacin, tetracycline and vancomycin.

9. The DNA oligonucleotide having a therapeutic effect on autoimmune diseases according to claim 6, wherein a base X in the nucleotide sequence set forth in SEQ ID NO:3 is an artificially produced base, and the artificially produced base is chemically modified with a medium-molecular-weight compound, a high-molecular-weight compound, a biopolymer, or a biocompatible polymer.

10. The DNA oligonucleotide having a therapeutic effect on autoimmune diseases according to claim 9, wherein the high-molecular-weight compound which modifies the artificially produced base is a polyethylene glycol (PEG) with a molecular weight of 20000 or more or any biocompatible large molecule with a molecular weight of 20000 or more.

11. The autoimmune disease therapeutic agent according to any one of claims 1 to 5, wherein the autoimmune disease to be treated is an autoimmune skin disease selected from the group consisting of alopecia areata, vitiligo vulgaris, psoriasis, scleroderma, dermatomyositis, atopic dermatitis, cutaneous lupus erythematosus and IgA-related dermatitis.

12. The autoimmune disease therapeutic agent according to any one of claims 1 to 5, wherein the autoimmune disease to be treated is an autoimmune disease in the bladder selected from the group consisting of interstitial cystitis and bladder pain syndrome.

13. The autoimmune disease therapeutic agent according to any one of claims 1 to 5, wherein the autoimmune disease to be treated is an autoimmune disease in the eye selected from the group consisting of uveitis, dry eye, keratitis sicca, episcleritis and scleritis.

14. The autoimmune disease therapeutic agent according to any one of claims 1 to 5, wherein the autoimmune disease to be treated is a systemic autoimmune disease selected from the group consisting of multiple sclerosis, systemic lupus erythematosus, endometriosis, myocarditis, type I diabetes mellitus, thyroiditis, premature ovarian failure, Sjogren syndrome, Raynaud syndrome, rheumatoid arthritis, myasthenia gravis, Takayasu arteritis, Addison disease, Guillain-Barre syndrome, hypothyroidism, sarcoidosis, hemophagocytic lymphohistiocytosis, thrombotic thrombocytopenic purpura, and autoimmune hepatitis.

15. The autoimmune disease therapeutic agent according to any one of claims 1 to 5, wherein the autoimmune disease to be treated is an autoimmune disease of the digestive organs selected from the group consisting of Crohn disease, ulcerative colitis, autoimmune pancreatitis, biliary cholangitis, and autoimmune atrophic gastritis.

16. An autoimmune disease therapeutic agent comprising as an active ingredient the DNA oligonucleotide according to any one of claims 6 to 10, wherein the autoimmune disease to be treated is an autoimmune skin disease selected from the group consisting of alopecia areata, vitiligo vulgaris, psoriasis, scleroderma, dermatomyositis, atopic dermatitis, cutaneous lupus erythematosus and IgA-related dermatitis.

17. An autoimmune disease therapeutic agent comprising as an active ingredient the DNA oligonucleotide according to any one of claims 6 to 10, wherein the autoimmune disease to be treated is an autoimmune disease in the bladder selected from the group consisting of interstitial cystitis and bladder pain syndrome.

18. An autoimmune disease therapeutic agent comprising as an active ingredient the DNA oligonucleotide according to any one of claims 6 to 10, wherein the autoimmune disease to be treated is an autoimmune disease in the eye selected from the group consisting of uveitis, dry eye, keratitis sicca, episcleritis and scleritis.

19. An autoimmune disease therapeutic agent comprising as an active ingredient the DNA oligonucleotide according to any one of claims 6 to 10, wherein the autoimmune disease to be treated is a systemic autoimmune disease selected from the group consisting of multiple sclerosis, systemic lupus erythematosus, endometriosis, myocarditis, type I diabetes mellitus, thyroiditis, premature ovarian failure, Sjogren syndrome, Raynaud syndrome, rheumatoid arthritis, myasthenia gravis, Takayasu arteritis, Addison disease, Guillain-Barre syndrome, hypothyroidism, sarcoidosis, hemophagocytic lymphohistiocytosis, thrombotic thrombocytopenic purpura, and autoimmune hepatitis.

20. An autoimmune disease therapeutic agent comprising as an active ingredient the DNA oligonucleotide according to any one of claims 6 to 10, wherein the autoimmune disease to be treated is an autoimmune disease of the digestive organs selected from the group consisting of Crohn disease, ulcerative colitis, autoimmune pancreatitis, biliary cholangitis, and autoimmune atrophic gastritis.
